(19) Europäisches Patentamt
European Patent Office
Office européen des brevets

(11) Veröffentlichungsnummer: **0 010 063**
**B1**

(12) # EUROPÄISCHE PATENTSCHRIFT

(45) Veröffentlichungstag der Patentschrift: **29.12.82**

(51) Int. Cl.³: **C 07 D 405/04,** C 07 D 413/04 // C07D235/12 ,(C07D405/04, 235/12, 307/85),(C07D413/04, 263/58, 307/85)

(21) Anmeldenummer: **79810104.4**

(22) Anmeldetag: **28.09.79**

(54) **Verfahren zur Herstellung von Furanyl-benzazolen.**

(30) Priorität: **04.10.78 CH 10304/78**

(43) Veröffentlichungstag der Anmeldung: **16.04.80 Patentblatt 80/8**

(45) Bekanntmachung des Hinweises auf die Patenterteilung: **29.12.82 Patentblatt 82/52**

(84) Benannte Vertragsstaaten: **CH DE FR GB IT**

(56) Entgegenhaltungen:
**DE-A-2 238 628**
**JOURNAL OF THE CHEMICAL SOCIETY, 1960, London, G. B. K. B. L. MATHUR et al.: »Preparation of 2-p-nitrophenylbenzofuran«, Seiten 1954, 1955**

(73) Patentinhaber: **CIBA-GEIGY AG, Patentabteilung Postfach, CH-4002 Basel (CH)**

(72) Erfinder: **Guglielmetti, Leonardo, Dr., Laufenburgerstrasse 30, CH-4058 Basel (CH)**
Erfinder: **Lüthi, Christian, Dr., Anwilerstrasse 10, CH-4059 Basel (CH)**

**EP 0 010 063 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von Furanyl-benzazolen

Die Erfindung betrifft ein Verfahren zur Herstellung von Furanyl-benzazolen.

Es sind bereits Verfahren zur Herstellung von 2-substituierten Benzofuranen bekanntgeworden, so z. B. (A) aus K. B. L. Mathur und H. S. Mehra, J. Chem. Soc. (London), 1960, 1954 – 1955, die Herstellung von 2-(p-Nitrophenyl)-benzofuran durch intramolekulare Kondensation von o-(p-Nitrobenzyloxy)-benzaldehyd; (B) aus der DE-OS 2 238 628 die Herstellung von 2-substituierten Benzofuranen durch Wasserabspaltung mit stark basischen Kondensationsmitteln aus o-Aralkoxy-carbonylverbindungen; (C) aus der DE-OS 2 361 338 die Herstellung durch Aminabspaltung aus o-aralkoxy-azomethinen durch stark basische Kondensationsmittel in stark polaren Lösungsmitteln und (D) aus der US-PS 3 772 323 die Herstellung von Benzofuranyl-benzimidazolen durch Kondensation einer Cumarilsäure mit einem N-mono-substituierten o-Phenylendiamin.

Alle diese Verfahren sind aber mit Nachteilen behaftet. Die aus (A) bekannte Reaktion ist nicht allgemein gültig; sie kann z. B. nicht mit o-Benzyloxybenzaldehyden durchgeführt werden, welche am Benzylrest keine Nitrogruppe besitzen und somit keine reaktionsfähigen Nitronsäuresalze ausbilden können. Die in (B) beschriebene Methode führt unter stark alkalischen Bedingungen zu unerwünschten Nebenreaktionen, welche die Ausbeute stark vermindern. Zur Behebung dieses Nachteils wurde ein anderer Syntheseweg gemäß (C) beschritten, welcher aber die unwirtschaftliche zusätzliche Stufe der Bildung und Isolierung eines Azomethins mit sich bringt und dazu noch den Nachteil der Rückgewinnung bzw. Beseitigung des anfallenden Amins aufweist. Der Nachteil der in (D) beschriebenen Methode liegt in der verhältnismäßig schweren Zugänglichkeit der Cumarilsäure.

Es wurde nun überraschenderweise gefunden, daß Furanylbenzazole ohne Isolierung von Zwischenstufen und in hoher Ausbeute durch Umsetzung von o-Hydroxycarbonylverbindungen mit 2-Halogenmethyl-benzazolen hergestellt werden können.

Das erfindungsgemäße Verfahren zur Herstellung von Furanyl-benzazolen der allgemeinen Formel 1

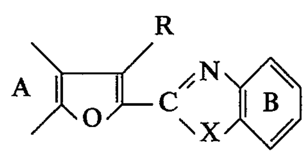

(1)

worin

A ein unsubstituiertes oder substituiertes carbocyclisches, aromatisches ein- oder mehrkerniges Ringsystem, das in der angegebenen Weise mit zwei benachbarten C-Atomen mit dem Furanring kondensiert ist,

R ein Wasserstoffatom, unsubstituiertes oder substituiertes Alkyl oder unsubstituiertes oder substituiertes Phenyl und

X ein Sauerstoffatom oder eine $-NR_5$-Gruppe bedeuten, worin $R_5$ für Alkenyl, Cycloalkyl, unsubstituiertes oder substituiertes Alkyl, Phenyl oder Aralkyl steht und

der Ring B unsubstituiert oder substituiert ist,

dadurch gekennzeichnet, daß man eine o-Hydroxycarbonylverbindung der allgemeinen Formel 2 mit einem 2-Halogenmethyl-benzazol der allgemeinen Formel 3

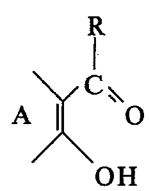

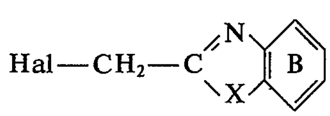

(2) (3)

worin A, B, R und X die oben angegebene Bedeutung haben und Hal für ein Halogenatom steht, in An- und Abwesenheit eines schwach basischen Kondensationsmittels bei Temperaturen von mindestens 50° C kondensiert.

Als schwach basische Kondensationsmittel kommen bestimmte anorganische und organische Verbindungen in Betracht, wie Alkali- und Erdalkalimetallverbindungen, z. B. Carbonate, Bicarbonate oder Acetate, Ammoniumverbindungen, wie Ammoniumacetat, oder tertiäre Amine, wie Pyridin. Vorzugsweise werden anorganische Verbindungen des Natriums und des Kaliums, besonders deren Carbonate, verwendet. Es können aber auch Gemische verschiedener schwach basischer Verbindungen verwendet werden.

Die einzusetzende Menge an Kondensationsmittel bewegt sich in weiten Grenzen. Obschon für das Gelingen der Reaktion an sich eine katalytische Menge ausreichend ist, verwendet man mit Vorteil

äquivalente Mengen oder sogar ein Vielfaches davon.

Das erfindungsgemäße Verfahren wird zweckmäßigerweise in einem unter den Reaktionsbedingungen inerten Lösungsmittel durchgeführt. Als solche Lösungsmittel kommen apolare und dipolare aprotische Lösungsmittel, wie Xylol, Dichlor- oder Trichlorbenzol, Dimethylformamid, Diäthylformamid, Dimethylacetamid, N-Methylpyrrolidon sowie deren Gemische in Betracht. Es werden vorzugsweise wasserfreie organische Lösungsmittel, worin die zur Anwendung gelangenden schwachen Basen teilweise oder vollständig löslich sind, verwendet.

In gewissen Fällen, z. B. wenn die Ausgangsstoffe tiefe Schmelzpunkte haben und sich nicht zersetzen, kann die erfindungsgemäße Umsetzung auch ohne Lösungsmittel, d. h. in der Schmelze, in Gegenwart des schwach basischen Kondensationsmittels durchgeführt werden.

Je nach Verfahren (mit oder ohne Lösungsmittel) und den zu kondensierenden Verbindungen kann sich die Reaktionstemperatur in einem breiten Bereich bewegen. Bei der Anwendung von Lösungsmitteln liegt sie zwischen 50°C und dem Siedepunkt des jeweiligen Lösungsmittels, vorzugsweise jedoch zwischen 50 und 200°C, besonders aber zwischen 90 und 160°C. Wird die Kondensation ohne Lösungsmittel durchgeführt, dann liegt die Reaktionstemperatur zwischen der Schmelztemperatur des Gemisches der verwendeten Reaktionskomponenten und der Zersetzungstemperatur der zu kondensierenden Verbindungen. Vorzugsweise kommen Temperaturen zwischen 100 und 250°C in Betracht.

Als Substituenten der erfindungsgemäß herstellbaren Furanyl-benzazole kommen solche in Betracht, welche die Kondensation nicht beeinträchtigen können, d. h. Substituenten, die mit den o-Hydroxycarbonylverbindungen der allgemeinen Formel 2 bzw. den 2-Halogenmethylverbindungen der allgemeinen Formel 3 nicht leicht reagieren können.

Eine wichtige Ausführungsform der Erfindung besteht in der Herstellung von Benzofuranyl-benzazolen der allgemeinen Formel 4

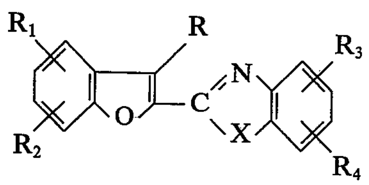

(4)

worin

R ein Wasserstoffatom oder unsubstituiertes oder nichtchromophor substituiertes Alkyl oder Phenyl,

$R_1$ ein Wasserstoffatom, Halogenatom, Alkenyloxy, Cycloalkoxy, unsubstituiertes oder nichtchromophor substituiertes Alkyl, Alkoxy, Phenoxy oder Aralkoxy, $-COOY_1$, $-CONY_1Y_2$ oder $-SO_2NY_1Y_2$, worin

$Y_1$ und $Y_2$ unabhängig voneinander jeweils für ein Wasserstoffatom, Alkenyl, Cycloalkyl mit 5 oder 6 Ring-C-Atomen oder unsubstituiertes oder nichtchromophor substituiertes Alkyl, Phenyl oder Aralkyl oder $Y_1$ und $Y_2$ zusammen mit dem Stickstoffatom für einen 5- oder 6gliedrigen gesättigten heterocyclischen Ring stehen, Mono- oder Dialkylamino, Acylamino, Sulfo, Arylsulfonyl, Alkylsulfonyl, Alkoxysulfonyl oder $R_1$ mit $R_2$ in o-Stellung zueinander den Rest $-CH=CH-CH=CH-$, $-O-CH_2-O-$ oder $-O-CH_2-CH_2-O-$ bilden,

$R_2$ ein Wasserstoffatom, Halogenatom oder unsubstituiertes oder nichtchromophor substituiertes Alkyl oder Alkoxy oder zusammen mit $R_1$ in o-Stellung zueinander den Rest $-CH=CH-CH=CH-$, $-O-CH_2-O-$ oder $-O-CH_2-CH_2-O-$ bilden,

$R_3$ ein Wasserstoffatom, Halogenatom, Alkenyloxy, Alkenylcarbonyl, unsubstituiertes oder nichtchromophor substituiertes Alkyl, Alkoxy, Phenyl oder Aralkyl, $-COOY_1$, $-CONY_1Y_2$ oder $-SO_2NY_1Y_2$, worin $Y_1$ und $Y_2$ die oben angegebene Bedeutung haben, Cyano, Sulfo, Alkylsulfonyl, Arylsulfonyl, Aryloxysulfonyl oder Trifluormethyl oder $R_3$ mit $R_4$ in o-Stellung zueinander den $-CH=CH-CH=CH$-Rest,

$R_4$ ein Wasserstoffatom, Halogenatom oder unsubstituiertes oder nichtchromophor substituiertes Alkyl oder Alkoxy oder $R_4$ mit $R_3$ in o-Stellung zueinander den Rest $-CH=CH-CH=CH-$ und

X ein Sauerstoffatom oder eine $-NR_5$-Gruppe, worin $R_5$ für Alkenyl, Cycloalkyl, unsubstituiertes oder nichtchromophor substituiertes Alkyl, Phenyl oder Aralkyl steht,

bedeuten, durch Kondensation einer o-Hydroxycarbonylverbindung der allgemeinen Formel 5 mit einem 2-Halogenmethyl-benzazol der allgemeinen Formel 6

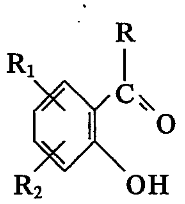

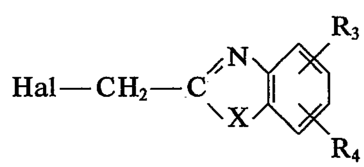

(5) (6)

worin R bis $R_4$ und X die oben angegebene Bedeutung haben und Hal ein Fluor-, Chlor- oder Bromatom bedeutet.

Alkylreste R, $R_1$, $R_2$, $R_3$, $R_4$ und $R_5$ haben 1 bis 8, vorzugsweise 1 bis 4 C-Atome und können als nichtchromophore Substituenten Hydroxy-, Cyano-, Alkoxy- mit vorzugsweise 1 bis 4 C-Atomen oder —COOZ-Gruppen, worin Z für ein Wasserstoffatom oder Alkyl mit vorzugsweise 1 bis 4 C-Atomen steht, aufweisen.

Ein Alkylrest $R_5$ kann auch durch einen Dialkylaminorest mit insgesamt 2 bis 6 C-Atomen substituiert sein.

Alkoxyreste $R_1$, $R_2$, $R_3$ und $R_4$ haben 1 bis 8, vorzugsweise 1 bis 4 C-Atome und als nichtchromophore Substituenten können sie Hydroxy-, nieder Alkoxy-, Carbonamido-, Cyano-, Alkoxycarbonyl- mit insgesamt 2 bis 5 C-Atomen oder Alkoxygruppen mit 1 bis 4 C-Atomen aufweisen.

Phenylreste R und $R_3$ sowie Phenoxyreste $R_1$ können als nichtchromophore Substituenten Halogenatome, wie Fluor-, Chlor- und Brom-, vorzugsweise Chloratome aufweisen oder Alkyl- oder Alkoxygruppen mit 1 bis 4, vorzugsweise 1 C-Atom besitzen.

Aralkoxyreste $R_1$ können im Arylrest, wie Phenylreste R, nichtchromophor substituiert sein und der Alkoxyteil weist 1 bis 4 C-Atome auf.

Alkylreste $R_5$, $Y_1$ und $Y_2$ können durch Hydroxy, nieder Alkoxy, Phenoxy, Carboxy, Carbalkoxy mit 2 bis 5 C-Atomen oder Cyano nichtchromophor substituiert sein.

Alkylreste in Alkylamino- und Alkylsulfonylgruppen haben 1 bis 8, vorzugsweise 1 bis 4 C-Atome.

Als Aralkylrest $R_5$ kommt besonders der Benzylrest in Betracht, der im Phenylteil, wie der Phenylrest R substituiert sein kann.

Alkoxysulfonylreste $R_3$ weisen 1 bis 4 C-Atome auf. Als Aryl- und Aryloxysulfonylreste $R_3$ kommen vorzugsweise der Phenyl- und Phenoxyrest in Betracht.

Unter Acylaminoreste $R_1$ sind besonders solche der Formel $-NH-COY_3$ zu verstehen, worin $Y_3$ für einen unsubstituierten oder substituierten Alkylrest mit 1 bis 8, vorzugsweise 1 bis 4 C-Atomen oder einen unsubstituierten oder substituierten Phenylrest steht.

Der Ausdruck »nieder« in Zusammenhang mit irgendeinem Rest bedeutet, daß der Rest 1 bis 4 C-Atome besitzt.

Bevorzugte Ausführungsformen des erfindungsgemäßen Verfahrens bestehen in der Herstellung von

I) Benzofuranyl-benzazolen der allgemeinen Formel 7

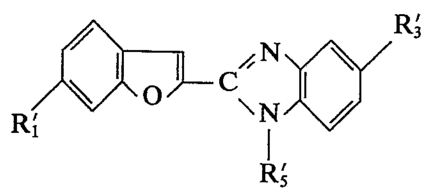

(7)

worin

$R_1'$ eine Alkoxygruppe mit 1 bis 8 Kohlenstoffatomen, eine Alkenyloxygruppe mit 3 oder 4 Kohlenstoffatomen, eine unsubstituierte oder durch Chlor, nieder Alkyl oder nieder Alkoxy substituierte Phenoxy- oder Phenylalkoxygruppe mit 1 bis 4 C-Atomen im Alkoxyteil, eine Hydroxyalkoxy- Alkoxyalkoxy-, Cyanoalkoxy-, Carbalkoxyalkoxy-, Carbonamidoalkoxy- oder Cyclohexyloxygruppe oder ein Wasserstoffatom,

$R_3'$ ein Wasserstoffatom, nieder Alkyl, nieder Alkoxy, ein Halogenatom, Phenyl, Alkylsulfonyl, Arylsulfonyl, unsubstituiertes oder ein- bis dreifach durch Alkyl mit 1 bis 4 C-Atomen, Chlor oder Methoxy substituiertes Phenoxysulfonyl, Cyano, Trifluormethyl, $-COOY_1$, $-SO_2NY_1Y_2'$ oder $-CONY_1Y_2'$, worin $Y_1$ für ein Wasserstoffatom, Alkyl mit 1 bis 8 C-Atomen, Alkenyl mit 3 oder 4 C-Atomen, Cyclohexyl, Hydroxyalkyl mit 2 bis 4 C-Atomen, Alkoxyalkyl mit insgesamt 3 bis 6 C-Atomen, Phenoxyalkyl mit insgesamt bis zu 9 C-Atomen, Carboxyalkyl mit 2 bis 6 C-Atomen, Carbalkoxyalkyl mit insgesamt 3 bis 6 C-Atomen, Cyanoalkyl mit 2 bis 5 C-Atomen, unsubstituiertes oder durch Methyl, Chlor oder Methoxy substituiertes Benzyl, unsubstituiertes oder durch Chlor, Methyl oder Methoxy substituiertes Phenyl, Dialkylaminoalkyl mit insgesamt 3 bis 7 C-Atomen oder Phenäthyl, $Y_2'$ für ein Wasserstoffatom, Alkyl mit 1 bis 4 C-Atomen, Alkenyl mit 3 oder 4 C-Atomen oder Hydroxyalkyl mit 2 bis 4 C-Atomen oder $Y_1$ und $Y_2'$ zusammen mit dem Stickstoffatom für einen 5- oder 6gliedrigen gesättigten heterocyclischen Ring stehen und

$R_5'$ Alkyl mit 1 bis 8 C-Atomen, Alkenyl mit 3 oder 4 C-Atomen, Cyclohexyl, Hydroxyalkyl mit 2 bis 4 C-Atomen, Alkoxyalkyl mit insgesamt 3 bis 6 C-Atomen, Carboxyalkyl mit 2 bis 5 C-Atomen, Carbalkoxyalkyl mit insgesamt 3 bis 9 C-Atomen, Cyanoalkyl mit 2 bis 5 C-Atomen, unsubstituiertes oder durch Chlor, Methyl oder Methoxy substituiertes Benzyl, Phenyl, Dialkylaminoalkyl mit insgesamt 3 bis 7 C-Atomen oder Phenäthyl bedeuten, durch Kondensation eines o-Hydroxybenzaldehyds der allgemeinen Formel 8 mit einem 2-Chlormethyl-benzimidazol der allgemeinen Formel 9

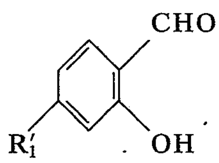 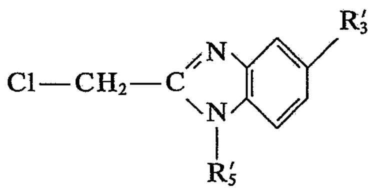

(8) (9)

worin $R'_1$, $R'_3$ und $R'_5$ die oben angegebene Bedeutung haben, und II) von Benzofuranyl-benzazolen der allgemeinen Formel 10

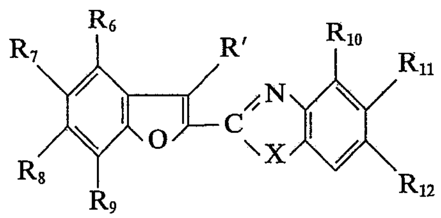

(10)

worin R′ ein Wasserstoffatom oder Methyl, $R_6$ ein Wasserstoffatom oder zusammen mit $R_7$ den Rest $-CH=CH-CH=CH-$, $R_7$ ein Wasserstoffatom, Chloratom, Alkyl mit 1 bis 4 C-Atomen, Alkylsulfonyl mit 1 bis 4, vorzugsweise 1 C-Atom oder zusammen mit $R_6$ den Rest $-CH=CH-CH=CH-$, $R_8$ ein Wasserstoffatom, Alkyl mit 1 bis 4, vorzugsweise 1 C-Atom, Alkoxy mit 1 bis 4 C-Atomen oder Dialkylamino mit insgesamt 2 bis 6, vorzugsweise 4 C-Atomen, $R_9$ ein Wasserstoffatom oder Chloratom, $R_{10}$ ein Wasserstoffatom oder zusammen mit $R_{11}$ den Rest $-CH=CH-CH=CH-$, $R_{11}$ ein Wasserstoffatom, Alkyl mit 1 bis 4, vorzugsweise 1 C-Atom, ein Chloratom, $-COOY_1'$ oder $-CONHY_1'$, worin $Y_1'$ für Alkyl mit 1 bis 4, vorzugsweise 1 bis 2 C-Atomen steht, $-CONH_2$, Cyano, Trifluormethyl, Sulfo, Alkylsulfonyl mit 1 bis 4, vorzugsweise 1 bis 2 C-Atomen, $-SO_2NHY_1'$ oder $-SO_2N(Y_1')_2$, worin $Y_1'$ die oben angegebene Bedeutung hat, $-SO_2NH_2$, Phenoxysulfonyl, Morpholinosulfonyl, Cyclohexylaminosulfonyl, Phenylaminosulfonyl oder zusammen mit $R_{10}$ den Rest $-CH=CH-CH=CH-$, $R_{12}$ ein Wasserstoffatom oder Phenyl und X ein Sauerstoffatom oder eine Gruppe $-NR_5''$, worin $R_5''$ für Alkyl mit 1 bis 8 C-Atomen, Hydroxyalkyl mit 2 bis 4, vorzugsweise 2 C-Atomen, Cyanoalkyl mit 2 bis 5, vorzugsweise 2 C-Atomen, unsubstituiertes oder durch Chlor oder Methoxy substituiertes Benzyl, Phenyl oder Cyclohexyl steht, bedeuten, wobei von $R_6$ bis $R_9$ eines oder zwei und von $R_{10}$ bis $R_{12}$ eines verschieden von Wasserstoff ist, durch Kondensation eines o-Hydroxy-benzaldehyds der allgemeinen Formel 11 mit einem 2-Chlormethyl-benzazol der allgemeinen Formel 12

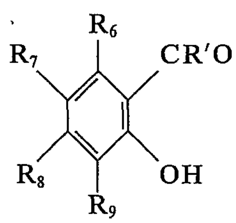 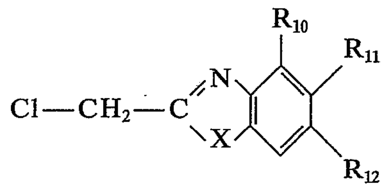

(11) (12)

worin R′ bis $R_{12}$ und X die oben angegebene Bedeutung haben.

Das erfindungsgemäße Verfahren ermöglicht die Herstellung von Furanyl-benzazolen in höheren Ausbeuten und ohne Isolierung von Zwischenprodukten. Die erfindungsgemäß herstellbaren Furanyl-benzazole sind Ausgangsprodukte für die Herstellung von pharmazentrischen Produkten (vgl. US-PS 3 470 192), Farbstoffen und Szintillatoren oder sind optische Aufheller (vgl. US-PS 3 772 323 und 4 009 994 oder die französische Patentschrift 2 359 839).

Die 2-Halogenmethylverbindungen der allgemeinen Formeln 3, 6, 9 und 12 können auch in Form derer Salze mit organischen oder anorganischen Säuren zur Anwendung gelangen.

Bei der Kondensation der Verbindungen der allgemeinen Formeln 2, 5, 8 und 11 mit solchen der allgemeinen Formeln 3, 6, 9 und 12 bilden sich intermediär die Äther der allgemeinen Formeln 13, 14, 15 und 16

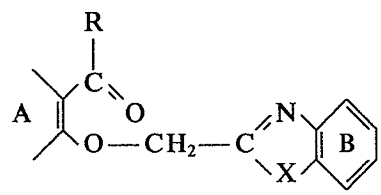

(13)

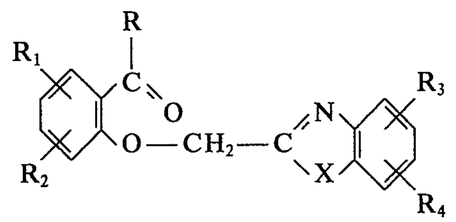

(14)

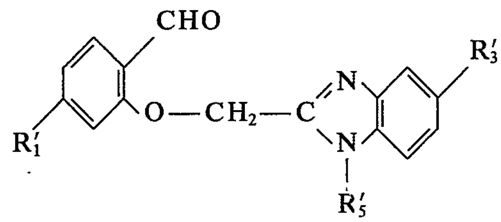

(15)

und

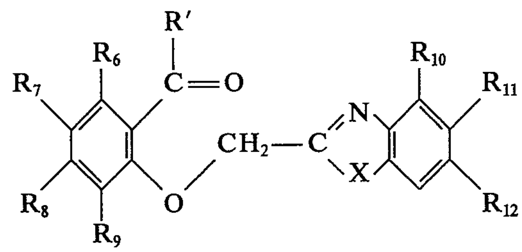

(16)

worin A, B, X, R bis $R_{12}$, R', $R_1'$, $R_3'$ und $R_5'$ die weiter oben angegebene Bedeutung haben. Diese Verbindungen sind neu und können vor dem Ringschluß als solche isoliert werden. Von besonderer Bedeutung sind jene der allgemeinen Formeln 14, 15 und 16.

Die nachfolgenden Beispiele erläutern die Erfindung. Prozente sind Gewichtsprozente.

## Beispiel 1

29,5 g 2-Chlormethyl-1-methyl-5-methylsulfonyl-benzimidazolhydrochlorid der Formel 100

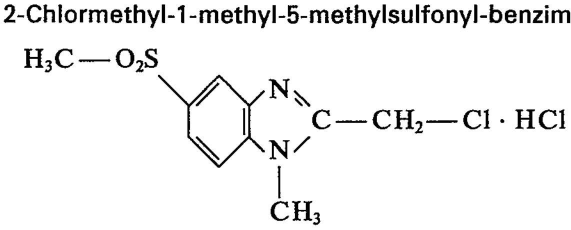

(100)

werden in 100 ml Dimethylformamid suspendiert. Die Suspension wird mit 16,3 g 2-Hydroxy-4-methoxy-benzaldehyd (Titer: 93,4%) der Formel 101

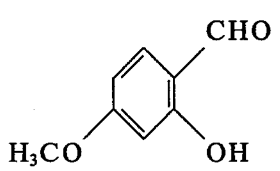

(101)

und 41,5 g wasserfreiem Kaliumcarbonat versehen. Das Reaktionsgemisch wird unter Stickstoff innerhalb 30 Minuten auf 90°C erhitzt und während einer Stunde bei dieser Temperatur weitergerührt, wobei der ziemlich unlösliche Äther der Formel 102

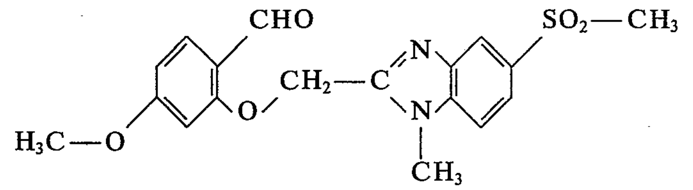

(102)

vom Fp. 204–206°C als Kondensationsprodukt ausfällt. Der dicke, aber immer noch gut rührbare Brei wird nun innerhalb 30 Minuten auf 140°C erhitzt, wobei unter Rühren und Stickstoff ab 130°C ein Gemisch von Wasser und Dimethylformamid langsam abdestilliert. Das Reaktionsgemisch wird nun während einer Stunde bei 140°C weitergerührt, wobei das durch den raschen Ringschluß des Kondensationsproduktes sich abspaltende Wasser ständig abdestilliert wird und das Reaktionsgemisch immer flüssiger wird. Das Reaktionsgemisch wird nun auf 30°C abgekühlt und bei dieser Temperatur unter Kühlung mit 200 ml Wasser langsam verdünnt, wobei das Reaktionsprodukt der Formel 103

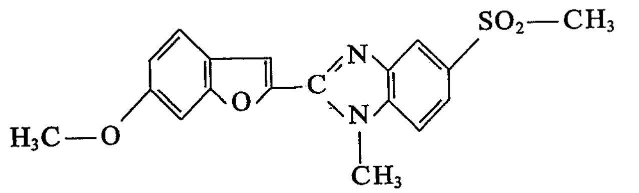

(103)

kristallin ausfällt. Das Reaktionsgemisch wird abgenutscht, das Nutschgut mit Wasser neutral gewaschen und unter Vakuum bei 80°C getrocknet. Man erhält 34,5 g (96% der Theorie) eines hellgelben kristallinen Pulvers vom Schmelzpunkt 193 bis 195°C. Ein analytisch reines aus Chlorbenzol umkristallisiertes Muster dieser Verbindung weist einen konstanten Schmelzpunkt von 196 bis 198°C auf.

Das als Ausgangsmaterial verwendete 2-Chlormethyl-1-methyl-5-methylsulfonyl-benzimidazolhydrochlorid der Formel 100 kann beispielsweise wie folgt hergestellt werden:

200,2 g 3-Amino-4-methylamino-phenylmethylsulfon und 99 g Chloressigsäure werden in 200 ml konzentrierter Salzsäure suspendiert, die Suspension auf Rückfluß (108°C) erhitzt, wobei bei 70°C eine klare Lösung entsteht. Das Reaktionsgemisch wird nach 10 Minuten Rückfluß mit einigen Kristallen 2-Chlormethyl-1-methyl-5-methylsulfonyl-benzimidazolhydrochlorid angeimpft und zwei Stunden am Rückfluß gehalten, wobei die Rückflußtemperatur von 108 auf 102°C abfällt und das Reaktionsprodukt kristallin ausfällt. Das kristalline breiartige Reaktionsgemisch wird nun auf 0 bis 5°C gekühlt, zwei Stunden bei dieser Temperatur weitergerührt und dann abgenutscht. Das Nutschgut wird mit 200 ml Isopropylalkohol gewaschen und unter Vakuum bei 80°C getrocknet. Man erhält 271 g (92% der Theorie) eines hellbraunen kristallinen Pulvers der Verbindung der Formel 100, die bei 247 bis 256°C unter Zersetzung schmilzt. Diese Verbindung ist dünnschichtchromatographisch rein und wird ohne Reinigung weiter eingesetzt.

## Beispiel 2

29,5 g des 2-Chlormethyl-1-methyl-5-methylsulfonyl-benzimidazolhydrochlorids der Formel 100 werden in 100 ml N-Methylpyrrolidon suspendiert. Die Suspension wird mit 16,3 g 2-Hydroxy-4-methoxy-benzaldehyd (Titer: 93,5%) und 41,5 g wasserfreiem Kaliumcarbonat versetzt. Die Kondensation wird wie im Beispiel 1 beschrieben durchgeführt. Das Reaktionsgemisch wird nachher unter Vakuum vom N-Methylpyrrolidon befreit und mit 200 ml Wasser langsam verdünnt, wobei das Reaktionsprodukt der Formel 103 kristallin ausfällt. Das Reaktionsprodukt wird nun abgenutscht, das Nutschgut mit Wasser neutral gewaschen und unter Vakuum bei 80°C getrocknet. Man erhält 34 g (95,5% der Theorie) eines hellgelben kristallinen Pulvers der Formel 103 vom Schmelzpunkt 196 bis 197°C.

## Beispiel 3

29,5 g des 2-Chlormethyl-1-methyl-5-methylsulfonyl-benzimidazolhydrochlorids der Formel 100 werden in 200 ml Chlorbenzol suspendiert. Die Suspension wird mit 16,3 g 2-Hydroxy-4-methoxy-benz-

aldehyd (Titer: 93,5%) und 41,5 g wasserfreiem Kaliumcarbonat versetzt. Das Reaktionsgemisch wird unter Stickstoff 20 Stunden am Rückfluß gehalten, wobei das sich abspaltende Wasser ständig entfernt wird. Das Reaktionsgemisch wird nun auf 30°C abgekühlt und bei dieser Temperatur unter Kühlung mit 300 ml Wasser langsam verdünnt, wobei das Reaktionsprodukt der Formel 103 kristallin ausfällt. Das Reaktionsgemisch wird unter Vakuum vom Chlorbenzol befreit, abgenutscht, das Nutschgut mit Wasser neutral gewaschen und unter Vakuum bei 80°C getrocknet. Man erhält 31,4 g (88,5% der Theorie) eines hellgelben kristallinen Pulvers der Formel 103 vom Schmelzpunkt 188 bis 193°C.

In analoger Weise werden die in der Tabelle I aufgeführten Verbindungen der allgemeinen Formel 300

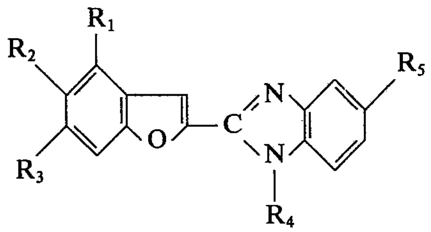

(300)

hergestellt.

Tabelle I

| Verbin-dung Nr. | $R_1$ | $R_2$ | $R_3$ | $R_4$ | $R_5$ | Schmelz-punkt, °C |
|---|---|---|---|---|---|---|
| 301 | $-H$ | $-H$ | $-O-CH_3$ | $-CH_2-$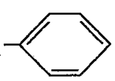 | $-SO_2-CH_3$ | 166 – 169 |
| 302 | $-H$ | $-Cl$ | $-O-CH_3$ | $-CH_2-$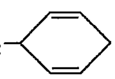 | $-SO_2-CH_3$ | 166 – 168 |
| 303 | $-H$ | $-H$ | $-O-CH_3$ | $-C_2H_5$ | $-SO_2-NH_2$ | 305 |
| 304 | $-H$ | $-H$ | $-O-C_4H_9(n)$ | $-CH_3$ | $-SO_2-CH_3$ | 195 – 196 |
| 305 | $-H$ | $-Cl$ | $-O-CH_3$ | $-CH_3$ | $-SO_2-CH_3$ | 278 |
| 306 | $-H$ | $-H$ | $-O-CH_3$ | $-C_4H_9(n)$ | $-SO_2-CH_3$ | 147 – 148 |
| 307 | $-H$ | $-H$ | $-O-CH_3$ | $-C_2H_5$ | $-SO_2-CH_3$ | 196 – 197 |
| 308 | $-H$ | $-H$ | $-O-CH_3$ | $-CH(CH_3)_2$ | $-SO_2-CH_3$ | 189 – 190 |
| 309 | $-H$ | $-H$ | $-O-CH_3$ | $-C_8H_{17}(n)$ | $-SO_2-CH_3$ | 137 – 138 |
| 310 | $-H$ | $-H$ | $-O-CH_3$ | $-CH_2-CH_2OH$ | $-SO_2-CH_3$ | 239 – 240 |
| 311 | $-H$ | $-H$ | $-O-CH_3$ | $-CH_3$ | $-SO_2-C_2H_5$ | 175 – 176 |
| 312 | $-H$ | $-H$ | $-O-CH_3$ | $-CH_3$ | $-SO_2-NH-CH_3$ | 243 |
| 313 | $-H$ | $-H$ | $-O-CH_3$ | $-CH_3$ | $-SO_2-NH_2$ | 339 (Zers.) |
| 314 | $-H$ | $-H$ | $-O-CH_3$ | $-CH_3$ | $-SO_3H$ | > 360 |
| 315 | $-H$ | $-H$ | $-O-CH_3$ | $-CH_3$ | $-SO_2-O-$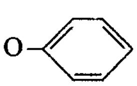 | 164 |
| 316 | $-H$ | $-H$ | $-O-CH_3$ | $-CH_3$ | $-SO_2-$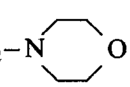 | 235 |

Fortsetzung

| Verbindung Nr. | $R_1$ | $R_2$ | $R_3$ | $R_4$ | $R_5$ | Schmelzpunkt, °C |
|---|---|---|---|---|---|---|
| 317 | $-H$ | $-H$ | $-O-CH_3$ | $-CH_2-$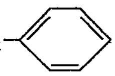 | $-SO_2N(CH_3)_2$ | 175 |
| 318 | $-H$ | $-H$ | $-O-CH_3$ | $-C_2H_5$ | $-SO_2NHC_2H_5$ | 217 |
| 319 | $-H$ | $-H$ | $-O-CH_3$ | 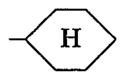 | $-SO_2NH-$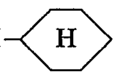 | 224 |
| 320 | $-H$ | $-H$ | $-O-CH_3$ | $-C_4H_9(n)$ | $-SO_2-NH-CH_3$ | 220 |
| 321 | $-H$ | $-H$ | $-O-CH_3$ | $-CH_3$ | $-SO_2NH-$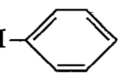 | 299 |
| 322 | $-H$ | $-H$ | $-N(C_2H_5)_2$ | $-CH_3$ | $-CH_3$ | 248 |

## Beispiel 4

25,6 g des 1-Benzyl-2-chlormethyl-benzimidazols der Formel 400

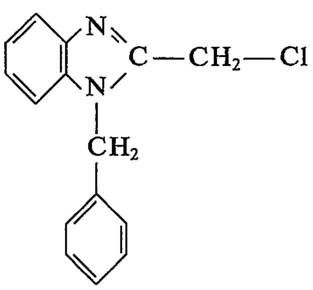

(400)

und 16,3 g 2-Hydroxy-4-methoxy-benzaldehyd (Titer: 93,5%) der Formel 102 werden in 100 ml Dimethylformamid aufgenommen, wobei eine klare Lösung entsteht. Zu dieser Lösung werden nun 27,6 g Kaliumcarbonat zugegeben. Das Reaktionsgemisch wird unter Stickstoff innerhalb 30 Minuten auf 90°C erhitzt und während einer Stunde bei dieser Temperatur weitergerührt. Die erhaltene gut rührbare rotbraune Suspension wird nun innerhalb 30 Minuten auf 150°C erhitzt, wobei unter Rühren und Stickstoff ab 130°C ein Gemisch von Wasser und Dimethylformamid langsam abdestilliert. Das Reaktionsgemisch wird nun während 6 Stunden bei 150°C weitergerührt, dann auf 0°C abgekühlt und bei dieser Temperatur mit 300 ml Wasser langsam verdünnt, wobei das Reaktionsprodukt der Formel 401

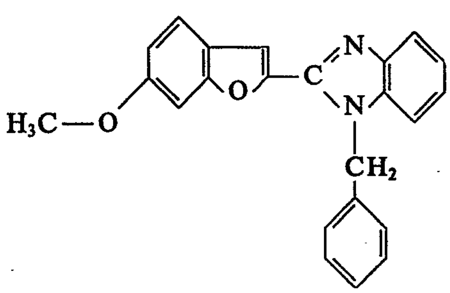

(401)

kristallin ausfällt. Das Reaktionsprodukt wird abgenutscht, das Nutschgut mit Wasser neutral gewaschen und unter Vakuum bei 80°C getrocknet. Man erhält 33,5 g (95% der Theorie) eines leicht braungefärbten kristallinen Pulvers vom Schmelzpunkt 132 bis 136°C. Ein analytisch reines aus Isopropylalkohol umkristallisiertes Muster dieser Verbindung weist einen konstanten Schmelzpunkt von 139 bis 140°C auf.

Das als Ausgangsmaterial verwendete 1-Benzyl-2-chlormethyl-benzimidazol der Formel 400 kann beispielsweise wie folgt hergestellt werden:

40 g N-Benzyl-1,2-phenylendiamin und 22 g Chloressigsäure werden in 60 ml konzentrierter Salzsäure suspendiert, die Suspension auf Rückfluß (108° C) erhitzt, wobei eine klare braunschwarze Lösung entsteht. Das Reaktionsgemisch wird nun zwei Stunden am Rückfluß gehalten, auf 10° C abgekühlt, mit 600 ml Methylenchlorid beschichtet und langsam unter Rühren mit 500 ml Wasser verdünnt. Die Methylenchlorid-Schicht wird abdekantiert, mit Wasser neutral gewaschen, mit Natriumsulfat getrocknet und zur Trockne eingeengt. Man erhält 44 g (86% der Theorie) einer hellbraunen kristallinen Masse der Verbindung der Formel 400, die bei 102 bis 106° C schmilzt. Diese Verbindung ist dünnschichtchromatographisch rein und wird ohne Reinigung weiter eingesetzt. Ein analytisch reines aus Äthanol umkristallisiertes Muster dieser Verbindung weist einen konstanten Schmelzpunkt von 108 bis 110° C auf.

Beispiel 5

Wenn man in Beispiel 4 das Dimethylformamid durch 200 ml 1,2-Dichlorbenzol ersetzt, erhält man 32,5 g (92% der Theorie) der Verbindung der Formel 401 als ein leicht braungefärbtes kristallines Pulver vom Schmelzpunkt 129 bis 133° C.

In analoger Weise werden die in der Tabelle II aufgeführten Verbindungen der allgemeinen Formel 500

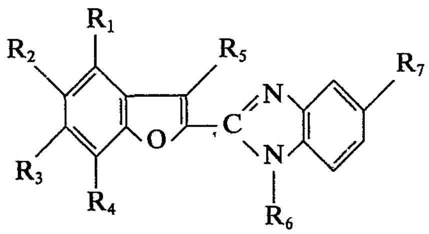

(500)

hergestellt.

Tabelle II

| Verbindung Nr. | $R_1$ | $R_2$ | $R_3$ | $R_4$ | $R_5$ | $R_6$ | $R_7$ | Fp., °C |
|---|---|---|---|---|---|---|---|---|
| 501 | $-H$ | $-H$ | $-O-CH_3$ | $-H$ | $-H$ | $-CH_3$ | $-H$ | 151 – 152 |
| 502 | $-H$ | $-H$ | $-O-CH_3$ | $-H$ | $-CH_3$ | $-CH_3$ | $-H$ | 159 – 160 |
| 503 | $-H$ | $-H$ | $-O-CH_3$ | $-H$ | $-H$ | 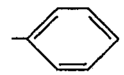 | $-H$ | 161 – 162 |
| 504 | 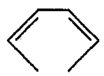 | | $-H$ | $-H$ | $-H$ | $-CH_3$ | $-H$ | 208 – 209 |
| 505 | $-H$ | $-H$ | $-CH_3$ | $-H$ | $-H$ | $-CH_2-$ 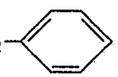 | $-H$ | 172 – 173 |
| 506 | $-H$ | $-Cl$ | $-H$ | $-Cl$ | $-H$ | $-CH_2-$ 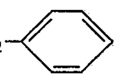 | $-H$ | 177 – 178 |
| 507 | $-H$ | $-H$ | $-O-CH_3$ | $-H$ | $-H$ | $-CH_2-$ 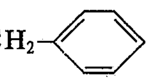 | $-CH_3$ | 157 – 159 |
| 508 | $-H$ | $-H$ | $-O-CH_3$ | $-H$ | $-H$ | $-CH_2-$ 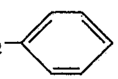 | $-Cl$ | 144 – 145 |
| 509 | $-H$ | $-SO_2CH_3$ | $-H$ | $-H$ | $-H$ | $-CH_2-$ 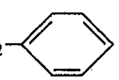 | $-H$ | 209 – 210 |
| 510 | $-H$ | $-H$ | $-OC_4H_9(n)$ | $-H$ | $-H$ | $-CH_2-$ 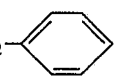 | $-H$ | 116 – 117 |

Fortsetzung

| Verbindung Nr. | $R_1$ | $R_2$ | $R_3$ | $R_4$ | $R_5$ | $R_6$ | $R_7$ | Fp., °C |
|---|---|---|---|---|---|---|---|---|
| 511 | 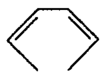 | | $-H$ | $-H$ | $-H$ | $-CH_2-$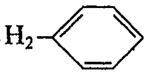 | $-H$ | 190 – 191 |
| 512 | $-H$ | $-H$ | $-O-CH_3$ | $-H$ | $-H$ | 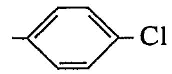$-Cl$ | $-H$ | 208 – 209 |
| 513 | $-H$ | $-H$ | $-O-CH_3$ | $-H$ | $-H$ | 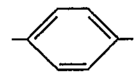$-OCH_3$ | $-H$ | 171 – 172 |
| 514 | $-H$ | $-H$ | $-O-CH_3$ | $-H$ | $-CH_3$ | 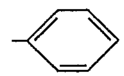 | $-H$ | 129 – 130 |
| 515 | $-H$ | $-H$ | $-O-CH_3$ | $-H$ | $-H$ | $-CH_2-CH_2-CN$ | $-H$ | 133 – 134 |
| 516 | $-H$ | $-C_2H_5$ | $-O-CH_3$ | $-H$ | $-H$ | $-CH_2-$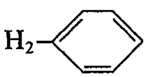 | $-H$ | 110 – 111 |
| 517 | $-H$ | $-H$ | $-O-CH_3$ | $-H$ | $-H$ | $-CH_3$ | $-COOC_2H_5$ | 203 – 204 |
| 518 | $-H$ | $-H$ | $-O-CH_3$ | $-H$ | $-H$ | $-CH_3$ | $-CONHCH_3$ | 251 – 252 |
| 519 | $-H$ | $-H$ | $-O-CH_3$ | $-H$ | $-H$ | $-CH_3$ | $-C\equiv N$ | 227 – 228 |
| 520 | $-H$ | $-H$ | $-O-CH_3$ | $-H$ | $-H$ | $-CH_3$ | $-CF_3$ | 150 – 151 |
| 521 | $-H$ | $-H$ | $-O-CH_3$ | $-H$ | $-H$ | $-CH_2-$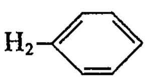 | $-CONH_2$ | 210 – 211 |
| 522 | $-H$ | $-H$ | $-O-CH_3$ | $-H$ | $-H$ | $-CH_3$ | $-COOCH_3$ | 199 – 200 |
| 523 | $-H$ | $-H$ | $-N(C_2H_5)_2$ | $-H$ | $-H$ | $-CH_3$ | $-H$ | 200 – 201 |

Beispiel 6

33,5 g des 2-Chlormethyl-benzoxazols der Formel 600

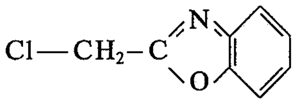

(600)

und 32,6 g 2-Hydroxy-4-methoxy-benzaldehyd (Titer: 93,6%) der Formel 101 werden in 100 ml Dimethylformamid aufgenommen, wobei eine klare Lösung entsteht. Zu dieser Lösung werden nun 55 g Kaliumcarbonat und 5 g Kaliumjodid zugegeben. Das Reaktionsgemisch wird unter Stickstoff innerhalb 30 Minuten auf 90° C erhitzt und während zwei Stunden bei dieser Temperatur weitergerührt. Die erhaltene gut rührbare braune Suspension wird nun innerhalb 30 Minuten auf 140° C erhitzt, wobei unter Rühren und Stickstoff ab 130° C ein Gemisch von Wasser und Dimethylformamid langsam abdestilliert. Das Reaktionsgemisch wird nun während zwei Stunden bei 140° C weitergerührt, dann auf 0° C abgekühlt und bei dieser Temperatur mit 300 ml Wasser langsam verdünnt, wobei das Reaktionsprodukt der Formel 601

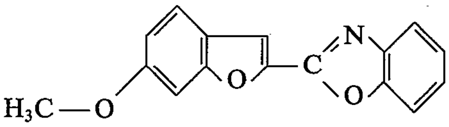

(601)

kristallin ausfällt. Das Reaktionsprodukt wird abgenutscht, das Nutschgut mit Wasser neutral gewaschen und unter Vakuum bei 80° C getrocknet. Man erhält 41,5 g (78% der Theorie) eines gelbgefärbten kristallinen Pulvers vom Schmelzpunkt 138 bis 143° C. Ein analytisch reines aus Nonan umkristallisiertes Muster dieser Verbindung weist einen konstanten Schmelzpunkt von 146 bis 147° C auf.

In analoger Weise werden die in der Tabelle III aufgeführten Verbindungen der allgemeinen Formel 602

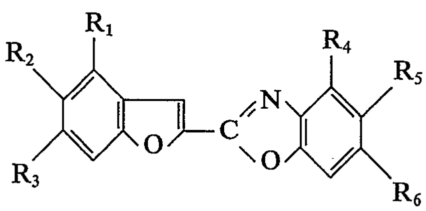

(602)

hergestellt.

Tabelle III

| Verbindung Nr. | $R_1$ | $R_2$ | $R_3$ | $R_4$ | $R_5$ | $R_6$ | Fp., °C |
|---|---|---|---|---|---|---|---|
| 603 | −H | −H | $-O-CH_3$ | −H | $-CH_3$ | −H | 128 − 129 |
| 604 | −H | −H | $-O-CH_3$ | −H | −H | 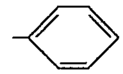 | 202 − 203 |
| 605 | −H | −H | $-O-CH_3$ | 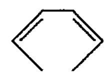 | | −H | 149 − 150 |
| 606 | −H | −H | −H | 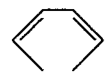 | | −H | 203 − 205 |
| 607 | −H | −H | −H | −H | 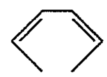 | | 298 − 300 |
| 608 | 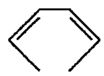 | | −H | −H | −H | −H | 172 − 173 |
| 609 | 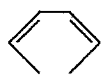 | | −H | −H | $-CH_3$ | −H | 194 − 195 |
| 610 | −H | −H | $-N(C_2H_5)_2$ | −H | −H | −H | 126 − 127 |

**Patentansprüche**

1. Verfahren zur Herstellung von Furanyl-benzazolen der allgemeinen Formel 1

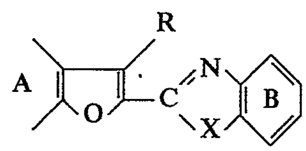

(1)

worin
A ein unsubstituiertes oder substituiertes carbocyclisches, aromatisches ein- oder mehrkerniges Ringsystem, das in der angegebenen Weise mit zwei benachbarten C-Atomen mit dem Furanring kondensiert ist,
R ein Wasserstoffatom, unsubstituiertes oder substituiertes Alkyl oder unsubstituiertes oder substituiertes Phenyl und
X Sauerstoff oder eine $-NR_5$-Gruppe bedeuten, worin $R_5$ für Alkenyl, Cycloalkyl, unsubstituiertes oder substituiertes Alkyl, Phenyl oder Aralkyl steht und
der Ring B unsubstituiert oder substituiert ist,
dadurch gekennzeichnet, daß man eine o-Hydroxycarbonylverbindung der allgemeinen Formel 2 mit einem 2-Halogenmethyl-benzazol der allgemeinen Formel 3

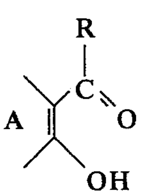

(2)

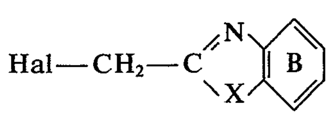

(3)

worin A, B, R und X die oben angegebene Bedeutung haben und Hal für ein Halogenatom steht, in An- oder Abwesenheit eines schwach basischen Kondensationsmittels bei Temperaturen von mindestens 50° C kondensiert.

2. Verfahren gemäß Anspruch 1, dadurch gekennzeichnet, daß man die Kondensation in Anwesenheit eines unter den Reaktionsbedingungen inerten organischen Lösungsmittels und eines schwach basischen Kondensationsmittels bei Temperaturen zwischen 50 und 200° C vornimmt.

3. Verfahren gemäß Anspruch 1, dadurch gekennzeichnet, daß man die Kondensation in Anwesenheit eines schwach basischen Kondensationsmittels und in Abwesenheit eines Lösungsmittels bei Temperaturen zwischen 100 und 250° C vornimmt.

4. Verfahren gemäß den Ansprüchen 1 bis 3 zur Herstellung von Benzofuranyl-benzazolen der allgemeinen Formel 4

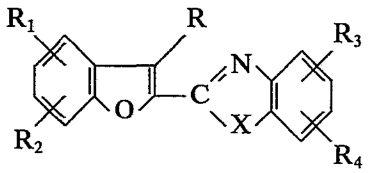

(4)

worin
R ein Wasserstoffatom oder unsubstituiertes oder nicht-chromophor substituiertes Alkyl oder Phenyl,
$R_1$ ein Wasserstoffatom, Halogenatom, Alkenyloxy, Cycloalkoxy, unsubstituiertes oder nicht-chromophor substituiertes Alkyl, Alkoxy, Phenoxy oder Aralkoxy, $-COOY_1$, $-CONY_1Y_2$ oder $-SO_2NY_1Y_2$, worin $Y_1$ und $Y_2$ unabhängig voneinander jeweils für ein Wasserstoffatom, Alkenyl, Cycloalkyl mit 5 oder 6 Ring C-Atomen oder unsubstituiertes oder nicht-chromophor substituiertes Alkyl, Phenyl oder Aralkyl oder $Y_1$ und $Y_2$ zusammen mit dem Stickstoffatom für einen 5- oder 6-gliedrigen gesättigten heterocyclischen Ring stehen, Mono- oder Dialkylamino, Acylamino, Sulfo, Arylsulfonyl, Alkylsulfonyl, Alkoxysulfonyl oder $R_1$ mit $R_2$ in o-Stellung zueinander den Rest $-CH=CH-CH=CH-$, $-O-CH_2-O-$ oder $-O-CH_2-CH_2-O-$ bilden,
$R_2$ ein Wasserstoffatom, Halogenatom oder unsubstituiertes oder nicht-chromophor substituiertes Alkyl oder Alkoxy oder zusammen mit $R_1$ in o-Stellung zueinander den Rest $-CH=CH-CH=CH-$, $-O-CH_2-O-$ oder $-O-CH_2-CH_2-O-$ bilden,
$R_3$ ein Wasserstoffatom, Halogenatom, Alkenyloxy, Alkenylcarbonyl, unsubstituiertes oder nicht-chromopher substituiertes Alkyl, Alkoxy, Phenyl oder Aralkyl, $-COOY_1$, $-CONY_1Y_2$ oder $-SO_2NY_1Y_2$, worin $Y_1$ und $Y_2$ die oben angegebene Bedeutung haben, Cyano, Sulfo, Alkylsulfonyl, Arylsulfonyl, Aryloxysulfonyl oder Trifluormethyl oder $R_3$ mit $R_4$ in o-Stellung zueinander den $-CH=CH-CH=CH$-Rest,
$R_4$ ein Wasserstoffatom, Halogenatom oder unsubstituiertes oder nicht-chromophor substituiertes Alkyl oder Alkoxy oder $R_4$ mit $R_3$ in o-Stellung zueinander den Rest $-CH=CH-CH=CH-$ und X ein Sauerstoffatom oder eine $-NR_5$-Gruppe, worin $R_5$ für Alkenyl, Cycloalkyl, unsubstituiertes oder nicht-chromophor substituiertes Alkyl, Phenyl oder Aralkyl steht,
bedeuten, durch Kondensation einer o-Hydroxycarbonylverbindung der allgemeinen Formel 5 mit einem 2-Halogenmethyl-benzazol der allgemeinen Formel 6

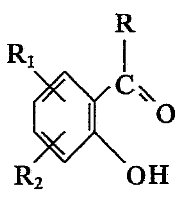

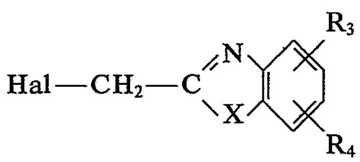

(5) (6)

worin R bis $R_4$ und X die oben angegebene Bedeutung haben und Hal ein Fluor-, Chlor- oder Bromatom bedeutet.

5. Verfahren gemäß den Ansprüchen 1 bis 3 zur Herstellung von Benzofuranyl-benzimidazolen der allgemeinen Formel 7

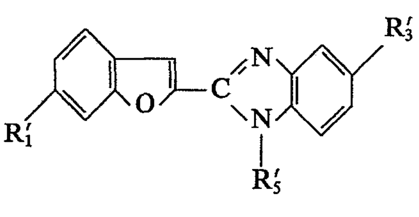

(7)

worin
$R'_1$ eine Alkoxygruppe mit 1 bis 8 Kohlenstoffatomen, eine Alkenyloxygruppe mit 3 oder 4

Kohlenstoffatomen, eine unsubstituierte oder durch Chor, nieder Alkyl oder nieder Alkoxy substituierte Phenoxy- oder Phenylalkoxygruppe mit 1 bis 4 C-Atomen im Alkoxyteil, eine Hydroxyalkoxy-, Alkoxyalkoxy-, Cyanoalkoxy-, Carbalkoxyalkoxy-, Carbonamidoalkoxy- oder Cyclohexyloxygruppe oder ein Wasserstoffatom,

$R'_3$ ein Wasserstoffatom, nieder Alkyl, nieder Alkoxy, ein Halogenatom, Phenyl, Alkylsulfonyl, Arylsulfonyl, unsubstituiertes oder ein bis dreifach durch Alkyl mit 1 bis 4 C-Atomen, Chlor oder Methoxy substituiertes Phenoxysulfonyl, Cyano, Trifluormethyl, $-COOY_1$, $-SO_2NY_1Y'_2$ oder $-CONY_1Y'_2$, worin $Y_1$ für ein Wasserstoffatom, Alkyl mit 1 bis 8 C-Atomen, Alkenyl mit 3 oder 4 C-Atomen, Cyclohexyl, Hydroxyalkyl mit 2 bis 4 C-Atomen, Alkoxyalkyl mit insgesamt 3 bis 6 C-Atomen, Phenoxyalkyl mit insgesamt bis zu 9 C-Atomen, Carboxyalkyl mit 2 bis 6 C-Atomen, Carbalkoxyalkyl mit insgesamt 3 bis 6 C-Atomen, Cyanoalkyl mit 2 bis 5 C-Atomen, unsubstituiertes oder durch Methyl, Chlor oder Methoxy substituiertes Benzyl, unsubstituiertes oder durch Chlor, Methyl oder Methoxy substituiertes Phenyl, Dialkylaminoalkyl mit insgesamt 3 bis 7 C-Atomen oder Phenäthyl, $Y'_2$ für ein Wasserstoffatom, Alkyl mit 1 bis 4 C-Atomen, Alkenyl mit 3 oder 4 C-Atomen oder Hydroxyalkyl mit 2 bis 4 C-Atomen oder $Y_1$ und $Y'_2$ zusammen mit dem Stickstoffatom für einen 5- oder 6-gliedrigen gesättigten heterocyclischen Ring stehen und

$R'_5$ Alkyl mit 1 bis 8 C-Atomen, Alkenyl mit 3 oder 4 C-Atomen, Cyclohexyl, Hydroxyalkyl mit 2 bis 4 C-Atomen, Alkoxyalkyl mit insgesamt 3 bis 6 C-Atomen, Carboxyalkyl mit 2 bis 5 C-Atomen, Carbalkoxyalkyl mit insgesamt 3 bis 9 C-Atomen, Cyanoalkyl mit 2 bis 5 C-Atomen, unsubstituiertes oder durch Chlor, Methyl oder Methoxy substituiertes Benzyl, Phenyl, Dialkylaminoalkyl mit insgesamt 3 bis 7 C-Atomen, oder Phenäthyl bedeuten, durch Kondensation eines o-Hydroxy-benzaldehyds der allgemeinen Formel 8 mit einem 2-Chlormethyl-benzimidazol der allgemeinen Formel 9

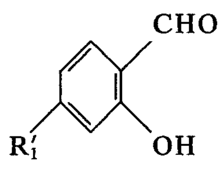

(8)

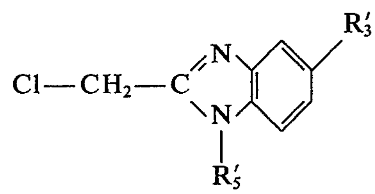

(9)

worin $R'_1$, $R'_3$ und $R'_5$ die oben angegebene Bedeutung haben.

6. Verfahren gemäß den Ansprüchen 1 bis 3 zur Herstellung von Benzofuranyl-benzazolen der allgemeinen Formel 10

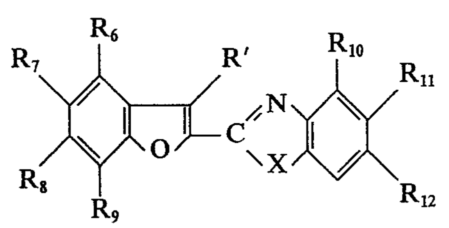

(10)

worin R' ein Wasserstoffatom oder Methyl, $R_6$ ein Wasserstoffatom oder zusammen mit $R_7$ den Rest $-CH=CH-CH=CH-$, $R_7$ ein Wasserstoffatom, Chloratom, Alkyl mit 1 bis 4 C-Atomen, Alkylsulfonyl mit 1 bis 4 C-Atomen oder zusammen mit $R_6$ den Rest $-CH=CH-CH=CH-$, $R_8$ ein Wasserstoffatom, Alkyl mit 1 bis 4 C-Atomen, Alkoxy mit 1 bis 4 C-Atomen oder Dialkylamino mit insgesamt 2 bis 6 C-Atomen, $R_9$ ein Wasserstoff- oder Chloratom, $R_{10}$ ein Wasserstoffatom oder zusammen mit $R_{11}$ den Rest $-CH=CH-CH=CH-$, $R_{11}$ ein Wasserstoffatom, Alkyl mit 1 bis 4 C-Atomen, ein Chloratom, $-COOY'_1$, oder $-CONHY'_1$, worin $Y'_1$ für Alkyl mit 1 bis 4 C-Atomen steht, $-CONH_2$, Cyano, Trifluormethyl, Sulfo, Alkylsulfonyl mit 1 bis 4 C-Atomen, $-SO_2NHY'_1$ oder $-SO_2N(Y'_1)_2$, worin $Y'_1$ die oben angegebene Bedeutung hat, $-SO_2NH_2$, Phenoxysulfonyl, Morpholinosulfonyl, Cyclohexylaminosulfonyl, Phenylaminosulfonyl oder zusammen mit $R_{10}$ den Rest $-CH=CH-CH=CH-$, $R_{12}$ ein Wasserstoffatom oder Phenyl und X ein Sauerstoffatom oder eine Gruppe $-NR''_5$, worin $R''_5$ für Alkyl mit 1 bis 8 C-Atomen, Hydroxyalkyl mit 2 bis 4 C-Atomen, Cyanoalkyl mit 2 bis 5 C-Atomen, unsubstituiertes oder durch Chlor oder Methoxy substituiertes Benzyl, Phenyl oder Cyclohexyl steht, bedeuten, wobei von $R_6$ bis $R_9$ eines oder zwei und von $R_{10}$ bis $R_{12}$ eines verschieden von Wasserstoff ist, durch Kondensation eines o-Hydroxy-benzaldehyds der allgemeinen Formel 11 mit einem 2-Chlormethylbenzazol der allgemeinen Formel 12

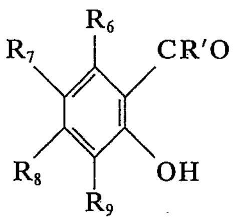 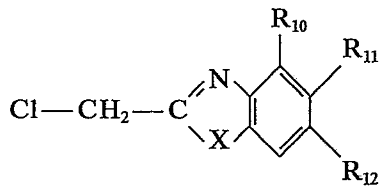

(11) (12)

worin R′ bis $R_{12}$ und X die oben angegebene Bedeutung haben.

7. Verfahren gemäß den Ansprüchen 1 bis 6, wobei als schwach basisches Kondensationsmittel Natrium- oder Kaliumcarbonat verwendet wird.

8. Verfahren gemäß den Ansprüchen 1 bis 7, wobei als unter den Reaktionsbedingungen inertes Lösungsmittel ein apolares oder dipolares aprotisches Lösungsmittel verwendet wird.

9. Verfahren gemäß Anspruch 8, wobei als Lösungsmittel Dimethylformamid verwendet wird.

## Claims

1. A process for the manufacture of furanylbenzazoles of the general formula (1)

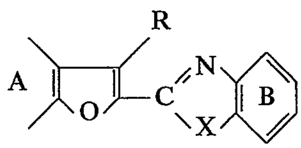

(1)

wherein A is an unsubstituted or substituted carbocyclic aromatic mono- or polynuclear ring system which is fused to the furan ring with two adjacent carbon atoms in the manner indicated, R is a hydrogen atom, unsubstituted or substituted alkyl or unsubstituted or substituted phenyl, X is oxygen or a $-NR_5$ group, in which $R_5$ is alkenyl, cycloalkyl, unsubstituted or substituted alkyl, phenyl or aralkyl, and the ring B is unsubstituted or substituted, which process comprises condensing an o-hydroxycarbonyl compound of the general formula (2)

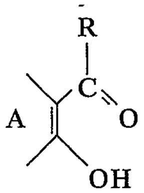

(2)

with a 2-halomethylbenzazole of the general formula (3)

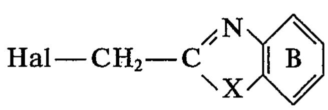

(3)

wherein A, B, R and X have the above meanings and Hal is a halogen atom, in the presence or absence of a weakly basic condensation agent, at a temperature of at least 50° C.

2. A process according to claim 1, wherein the condensation is carried out in the presence of an organic solvent which is inert under the reaction conditions and of a weakly basic condensation agent in the temperature range from 50° to 200° C.

3. A process according to claim 1, wherein the condensation is carried out in the presence of a weakly basic condensation agent and in the absence of a solvent in the temperature range between 100° and 250° C.

4. A process according to claims 1 to 3 for the manufacture of benzofuranylbenzazoles of the general formula (4)

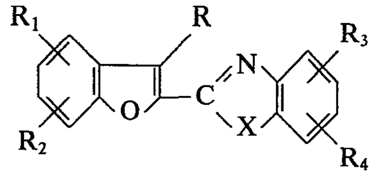

(4)

wherein R is a hydrogen atom or alkyl or phenyl which are unsubstituted or substituted by non-chromophoric groups; $R_1$ is a hydrogen atom, a halogen atom, alkenyloxy, cycloalkoxy, or alkyl, alkoxy, phenoxy or aralkoxy which are unsubstituted or substituted by non-chromophoric groups, $-COOY_1$, $-CONY_1Y_2$ or $-SO_2NY_1Y_2$, in which $Y_1$ and $Y_2$, each independently of the other, are a hydrogen atom, alkenyl, cycloalkyl containing 5 or 6 ring carbon atoms, or alkyl, phenyl or aralkyl which are unsubstituted or substituted by non-chromophoric groups, or $Y_1$ and $Y_2$ together with the nitrogen atom to which they are attached form a 5- or 6-membered saturated heterocyclic ring, or $R_1$ is mono- or dialkylamino, acylamino, sulfo, arylsulfonyl, alkylsulfonyl, alkoxysulfonyl, or $R_1$ and $R_2$ in the orthoposition to each other form the radical $-CH=CH-CH=CH-$, $-O-CH_2-O-$ or $-O-CH_2-CH_2-O-$; $R_2$ is a hydrogen atom, a halogen atom, or alkyl or alkoxy which are unsubstituted or substituted by non-chromophoric groups, or together with $R_1$ in the ortho-position to each other forms the radical $-CH=CH-CH=CH-$, $-O-CH_2-O-$ or $-O-CH_2-CH_2-O-$; $R_3$ is a hydrogen atom, a halogen atom, alkenyloxy, alkenylcarbonyl, or alkyl, alkoxy, phenyl or aralkyl which are unsubstituted or substituted by non-chromophoric groups, or is $-COOY_1$, $-CONY_1Y_2$ or $-SO_2NY_1Y_2$, in which $Y_1$ and $Y_2$ have the above meanings, or is cyano, sulfo, alkylsulfonyl, arylsulfonyl, aryloxysulfonyl or trifluoromethyl, or $R_3$ and $R_4$ in the ortho-position to each other form the radical $-CH=CH-CH=CH-$; $R_4$ a hydrogen atom, a halogen atom, or alkyl or alkoxy which are unsubstituted or substituted by non-chromophoric groups, or $R_4$ nad $R_3$ in the ortho-position to each other form the radical $-CH=CH-CH=CH-$ and X is an oxygen atom or a $-NR_5$ group, in which $R_5$ is alkenyl, cycloalkyl, or alkyl, phenyl or aralkyl which are unsubstituted or substituted by non-chromophoric groups, which process comprises condensing an o-hydroxycarbonyl compound of the general formula (5)

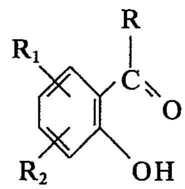

(5)

with a 3-halomethylbenzazole of the formula (6)

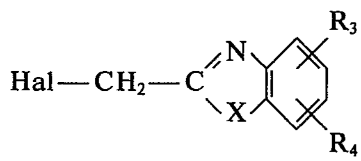

(6)

wherein R to $R_4$ and X are as defined above and Hal is a fluorine, chlorine or bromine atom.

5. A process according to claims 1 to 3 for the manufacture of benzofuranylbenzazoles of the formula

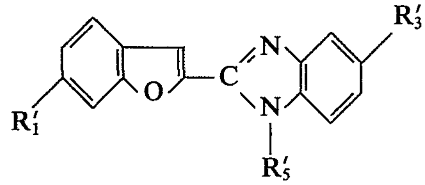

(7)

wherein $R'_1$ is an alkoxy group of 1 to 8 carbon atoms, an alkenyloxy group of 3 to 4 carbon atoms, a phenoxy or phenylalkoxy group containing 1 to 4 carbon atoms in the alkoxy moiety and which is unsubstituted or substituted by chlorine, lower alkyl or lower alkoxy, a hydroxyalkyloxy, alkoxyalkoxy, cyanoalkoxy, carbalkoxyalkoxy, carbonylalkoxy or cyclohexyloxy group or a hydrogen atom; $R'_3$ is a hydrogen atom, lower alkyl, lower alkoxy, a halogen atom, phenyl, alkylsulfonyl, arylsulfonyl, phenoxysulfonyl which is unubstituted or mono- to trisubstituted by alkyl of 1 to 4 carbon atom, chlorine or methoxy, or is cyano, trifluoromethyl, $-COOY_1$, $-SO_2NY_1Y'_2$ or $-CONY_1Y'_2$, wherein $Y_1$ is a

hydrogen atom, alkyl of 1 to 8 carbon atoms, alkenyl of 3 or 4 carbon atoms, cyclohexyl, hydroxyalkyl of 2 to 4 carbon atoms, alkoxyalkyl containing a total of 3 to 6 carbon atoms, phenoxyalkyl containing a total of 6 to 9 carbon atoms, carboxyalkyl of 2 to 6 carbon atoms, carbalkoxyalkyl containing a total of 3 to 6 carbon atoms, cycloalkyl of 2 to 5 carbon atoms, benzyl which is unsubstituted or substituted by methyl, chlorine or methoxy, phenyl which is unsubstituted or substituted by chlorine, methyl or methoxy, dialkylaminoalkyl containing a total of 3 to 7 carbon atoms or phenethyl, $Y'_2$ is a hydrogen atom, alkyl of 1 to 4 carbon atoms, alkenyl of 3 or 4 carbon atoms or hydroxyalkyl of 2 to 4 carbon atoms, or $Y_1$ and $Y'_2$ together with the nitrogen atom to which they are attached form a 5- or 6-membered saturated heterocyclic ring; and $R'_5$ is alkyl of 1 to 8 carbon atoms, alkenyl of 3 to 4 carbon atoms, cyclohexyl, hydroxyalkyl of 2 to 4 carbon atoms, alkoxyalkyl containing a total of 3 to 6 carbon atoms, carboxyalkyl of 2 to 5 carbon atoms, carbalkoxyalkyl containing a total of 3 to 9 carbon atoms, cyanoalkyl of 2 to 5 carbon atoms, benzyl which is unsubstituted or substituted by chlorine, methyl or methoxy, or is phenyl, dialkylaminoalkyl containing a total of 3 to 7 carbon atoms or phenethyl, which process comprises condensing an o-hydroxylbenzaldehyde of the general formula (8)

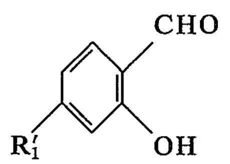

(8)

with a 2-chloromethylbenzimidazole of the general formula (9)

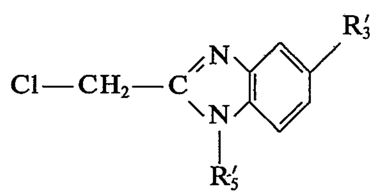

(9)

wherein $R'_1$, $R'_3$ and $R'_5$ are defined above.

6. A process according to claims 1 to 3 for the manufacture of benzofuranylbenzazoles of the general formula (10)

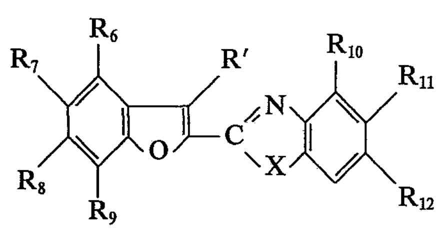

(10)

wherein R′ is a hydrogen atom or methyl, $R_6$ is a hydrogen atom or together with $R_7$ is the radical $-CH=CH-CH=CH-$, $R_7$ is a hydrogen atom, a chlorine atom, alkyl of 1 to 4 carbon atoms, alkylsulfonyl of 1 to 4 carbon atoms, or together with $R_6$ is the radical $-CH=CH-CH=CH-$, $R_8$ is a hydrogen atom, alkyl of 1 to 4 carbon atoms, alkoxy of 1 to 4 carbon atoms or dialkylamino containing a total of 2 to 6 carbon atoms, $R_9$ is a hydrogen atom or a chlorine atom, $R_{10}$ is a hydrogen atom or together with $R_{11}$ is the radical $-CH=Ch-CH=CH-$, $R_{11}$ is a hydrogen atom, alkyl of 1 to 4 carbon atoms, a chlorine atom, $-COOY'_1$ or $-CONHY'_1$, wherein $Y'_1$ is alkyl of 1 to 4 carbon atoms, $-CONH_2$, cyano, trifluoromethyl, sulfo, alkylsulfonyl of 1 to 4 carbon atoms, $-SO_2NHY'_1$ or $-SO_2N(Y'_1)_2$, wherein $Y'_1$ is as defined above, $-SO_2NH_2$, phenoxysulfonyl, morpholinosulfonyl, cyclohexylaminosulfonyl, phenylaminosulfonyl or together with $R_{10}$ is the radical $-CH=CH-CH=CH-$, $R_{12}$ is a hydrogen atom or phenyl and X is an oxygen atom or a group $-NR''_5$, wherein $R''_5$, is alkyl of 1 to 8 carbon atoms, hydroxyalkyl of 2 to 4 carbon atoms, cyanoalkyl of 2 to 5 carbon atoms, benzyl which is unsubstituted or substituted by chlorine or methoxy or is phenyl or cyclohexyl, with the proviso that one or two of $R_6$ to $R_9$ and one of $R_{10}$ to $R_{12}$ is different from hydrogen, which process comprises condensing an o-hydroxybenzaldehyde of the general formula

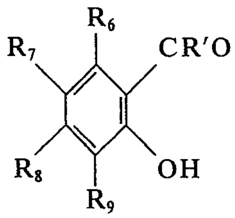

(11)

with a 2-chloromethylbenzazole of the general formula (12)

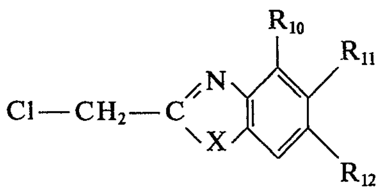

(12)

wherein R' to $R_{12}$ and X are as defined above.

7. A process according to claims 1 to 6, wherein the weakly basic condensation agent is sodium or potassium carbonate.

8. A process according to claims 1 to 7, wherein the solvent which is inert under the raction conditions is an apolar or a dipolar aprotic solvent.

9. A process according to claim 8, wherein the solvent is dimethyl formamide.

**Revendications**

1. Procédé de préparation de furanyl-benzazoles de formule générale (1):

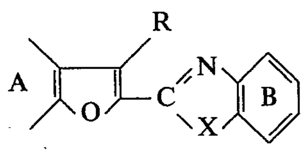

(1)

où
A désigne un système carbocyclique ou aromatique à 1 ou plusieurs noyaux non substitués ou substitués, que l'on condense de la façon indiquée avec le noyau furanique par deux atomes de carbone contigüs,
R désigne un atome d'hydrogène, un alkyle substitué ou non substitué, un phényle substitué ou non substitué et
X désigne de l'oxygène ou un groupe $-NR_5$ où $R_5$ désigne alkényle, cyclo-alkyle, alkyle, phényle ou aralkyle non substitués ou substitués, et le noyau B est non substitué ou substitué, caractérisé par le fait qu'on condense un composé o-hydroxycarbonyle de formule générale (2) avec un 2-halogénométhyl-benzazol de formule générale (3):

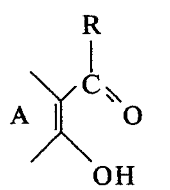

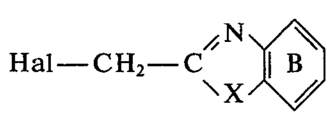

(2) (3)

où A, B, R et X ont les significations ci-dessus indiquées et Hal désigne un atome d'halogène, en l'absence ou en présence d'un agent de condensation faiblement basique, à des températures d'au moins de 50° C.

2. Procédé selon la revendication 1, caractérisé par le fait que le condensation est mise en oeuvre en présence d'un solvant organique inerte dans les conditions de la réaction et en présence d'un agent de condensation faiblement basique, à des températures comprises entre 50 et 200° C.

3. Procédé selon la revendications 1, caractérisé par le fait que la condensation est mise en oeuvre en présence d'un agent de condensation faiblement basique basique et en l'absence d'un solvant, à

des températures comprises entre 100 et 250° C.

4. Procédé selon les revendications 1 à 3 pour la préparation de benzofuranyl-benzazoles de formule générale (4):

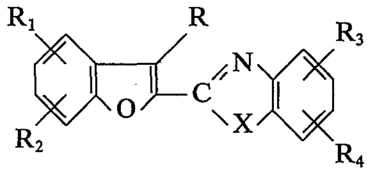

(4)

où

R désigne un atome d'hydrogène ou un alkyle ou phényle non substitués ou substitués par un non-chromophore,

$R_1$ désigne un atome d'hydrogène, un atome d'halogène, alkényloxy, cyclo-alcoxy ou bien alkyle, alcoxy, phénoxy ou aralcoxy non substitués ou substitués par un non-chromophore; $-COOY_1$, $-CONY_1Y_2$ ou $-SO_2NY_1Y_2$ où $Y_1$ et $Y_2$ identiques ou différents désignent un atome d'hydrogène, alkényle, cyclo-alkyle ayant 5 ou 6 atomes de carbone dans le noyau ou bien alkyle, phényle ou aralkyle non substitués ou substitués par un chromophore ou bien $Y_1$ et $Y_2$ forment ensemble avec l'atome d'azote un hétérocycle saturé à 5 ou 6 chaînons, mono- ou dialkylamino, acylamino, sulfo, arylsulfonyle, alkylsulfonyle, alcoxysulfonyle où $R_1$ et $R_2$ en position o l'un par rapport à l'autre forment le reste $-CH=CH-CH=CH-$, $-O-CH_2-O-$ ou $-O-CH_2-CH_2-O-$;

$R_2$ désigne un atome d'hydrogène, un atome d'halogène ou un alkyle ou alcoxy non substitués ou substitués par un non-chromophore ou bien forment ensemble avec $R_1$ en position o l'un par rapport à l'autre le reste $-CH=CH-CH=CH-$, $-O-CH_2-O-$ ou $-O-CH_2-CH_2-O-$;

$R_3$ désigne un atome d'hydrogène, un atome d'halogène, alkényloxy, alkénylcarbonyle, alkyle, alcoxy, phényle ou aralkyle non substitués ou substitués par un non-chromophore, $-COOY_1$, $-CONY_1Y_2$ ou $-SO_2NY_1Y_2$, ou $Y_1$ et $Y_2$ ont la signification cidessus indiquée, cyano, sulfo, alkylsulfonyle, arylsulfonyle, aryloxysulfonyle ou trifluorométhyle ou bien $R_3$ et $R_4$ en position o l'un par rapport à l'autre forment le reste $-CH=CH-CH=CH$;

$R_4$ désigne un atome d'hydrogène, un atome d'halogène ou bien un alkyle ou alcoxy non substitué ou substitué par un non-chromophore ou bien $R_4$ et $R_3$ en position o l'un par rapport à l'autre forment le reste $-CH=CH-CH=CH-$ et X désigne un atome d'oxygène ou un groupe $-NR_5$ où $R_5$ désigne alkényle, cyclo-alkyle ou un alkyle, phényle ou aralkyle non substitués ou substitués par un non-chromophore, par condensation d'un composé o-hydroxycarbonyle de formule générale (5) avec un 2-halogénométhylbenzazole de formule générale (6):

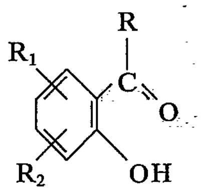

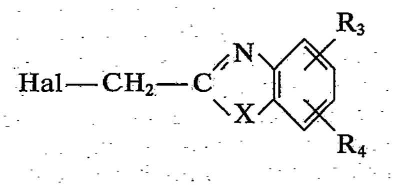

(5) (6)

où R à $R_4$ et X ont la signification ci-dessus indiquée et Hal désigne un atome de fluore, de chlore ou de brome.

5. Procédé selon les revendications 1 à 3 pour la préparation de benzofuranyl-benzimidazoles de formule générale (7):

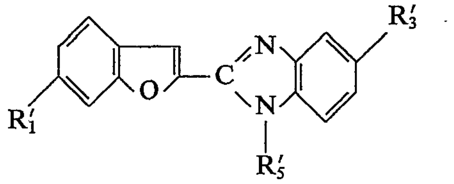

(7)

où

$R'_1$ désigne un groupe alcoxy ayant de 1 à 8 atomes de carbone, un groupe alkényloxy ayant 3 ou 4 atomes de carbone, un groupe phénoxy ou phénylalcoxy ayant de 1 à 4 atomes de carbone dans la partie alcoxy, non substitués ou substitués par chlore, alkyle inférieur ou alcoxy inférieur; un groupe hydroxyalcoxy, alcoxyalcoxy, cyano-alcoxy, carbalcoxyalcoxy, carbonamido-alcoxy ou cyclohexyloxy

ou un atome d'hydrogène,

R'$_3$ désigne un atome d'hydrogène, alkyle inférieur, alcoxy inférieur, un atome d'halogène, phényle, alkylsulfonyle, arylsulfonyle ou phénoxysulfonyle non substitué ou substitué 1 à 3 fois par alkyle ayant de 1 à 4 atomes de carbone, par chlore ou par méthoxy; cyano, trifluorométhyle, $-COOY_1$, $-SO_2NY_1Y'_2$ ou $-CONY_1Y'_2$ où $Y_1$ désigne un atome d'hydrogène, alkyle ayant de 1 à 8 atomes de carbone, alkényle ayant 3 ou 4 atomes de carbone, cylohexyle, hydroxyalkyle ayant 2 à 4 atomes de carbone, alcoxyalkyle ayant au total 3 à 6 atomes de carbone, phénoxyalkyle ayant au total jusqu'à 9 atomes de carbone, carboxyalkyle ayant 2 à 6 atomes de carbone, carbalcoxyalkyle ayant au total 3 à 6 atomes de carbone, cyano-alkyle ayant 2 à 5 atomes de carbone, benzyle non substitué ou substitué par méthyle, chlore ou méthoxy, phényle non substitué ou substitué par chlore, méthyle ou méthoxy, dialkylamino-alkyle ayant au total 3 à 7 atomes de carbone ou phénéthyle, $Y'_2$ désigne un atome d'hydrogène, alkyle ayant 1 à 4 atomes de carbone, alkényle ayant 3 ou 4 atomes de carbone ou hydroxyalkyle ayant 2 à 4 atomes de carbone ou bien $Y_1$ et $Y'_2$ forment ensemble avec l'atome d'azote un noyau hétérocyclique saturé ayant 5 à 6 chaînons, et

R'$_5$ désigne alkyle ayant 1 à 8 atomes de carbone, alkényle ayant 3 ou 4 atomes de carbone, cyclohexyle, hydroxyalkyle, ayant 2 à 4 atomes de carbone, alcoxyalkyle ayant au total 3 à 6 atomes de carbone, carboxyalkyle ayant 2 à 5 atomes de carbone, carbalcoxyalkyle ayant au total 3 à 9 atomes de carbone, cyanoalkyle ayant 2 à 5 atomes de carbone, benzyle ou phényle non substitués ou substitués par chlore, méthyle ou méthoxy, dialkylamino-alkyle ayant au total 3 à 7 atomes de carbone ou phénéthyle, par condensation d'un o-hydroxybenzaldéhyde de formule générale (8) avec un 2-chlorométhylbenzimidazol de formule générale (9):

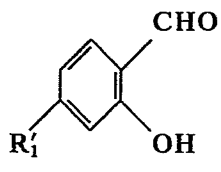

(8)

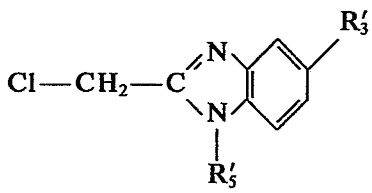

(9)

où $R'_1$, $R'_3$ et $R'_5$ ont les significations ci-dessus indiquées.

6. Procédé selon les revendications 1 à 3 pour la préparation de benzofuranyl-benzazoles de formule générale (10)

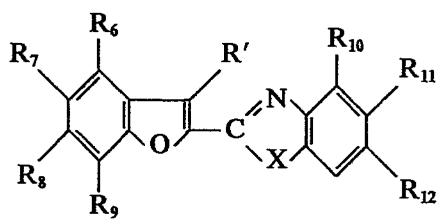

(10)

où R' désigne un atome d'hydrogène ou méthyle, $R_6$ désigne un atome d'hydrogène ou forme avec $R_7$ le reste $-CH=CH-CH=CH-$,

$R_7$ désigne un atome d'hydrogène, un atome de chlore, alkyle ayant de 1 à 4 atomes de carbone, alkylsulfonyle ayant 1 à 4 atomes de carbone ou bien forme avec $R_6$ le reste $-CH=CH-CH=CH-$,

$R_8$ désigne un atome d'hydrogène, alkyle ayant de 1 à 4 atomes de carbone, alcoxy ayant de 1 à 4 atomes de carbone ou dialkylamino ayant au total 2 à 6 atomes de carbone, $R_9$ désigne un atome d'hydrogène ou de chlore, $R_{10}$ désigne un atome d'hydrogène ou bien forment ensemble avec $R_{11}$ le reste $-CH=CH-CH=CH-$, $R_{11}$ désigne un atome d'hydrogène, alkyle ayant de 1 à 4 atomes de carbone, un atome de chlore, $-COOY'_1$ ou $-CONHY'_1$ où $Y'_1$ désigne alkyle ayant 1 à 4 atomes de carbone, $-CONH_2$, cyano, trifluorométhyle, sulfo, alkylsulfonyle ayant de 1 à 4 atomes de carbone, $-SO_2NHY'_1$ ou $-SO_2N(Y'_1)_2$, où $Y'_1$ a la signification ci-dessus indiquée, $-SO_2NH_2$, phénoxysulfonyle, morpholinosulfonyle, cyclohexylaminosulfonyle, phénylaminosulfonyle ou bien forment avec $R_{10}$ le reste $-CH=CH-CH=CH-$, $R_{12}$ désigne un atome d'hydrogène ou phényle et X désigne un atome d'oxygène ou un groupe $-NR''_5$ où $R''_5$ désigne alkyle ayant 1 à 8 atomes de carbone, hydroxyalkyle ayant 2 à 4 atomes de carbone, cyano-alkyle ayant 2 à 5 atomes de carbone, benzyle, phényle, non substitués ou substitués par chlore ou méthoxy, ou cyclohexyle, un ou deux des symboles $R_6$ à $R_9$ et l'un des symboles $R_{10}$ à $R_{12}$ est différent d'un atome d'hydrogène, par condensation d'un o-hydroxybenzaldéhyde de formule générale (11) avec un 2-chlorométhylbenzazole de formule générale (12):

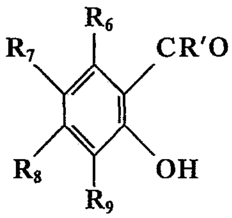

(11)

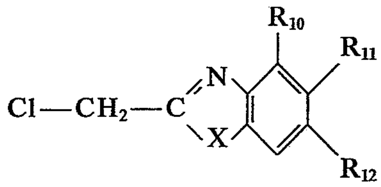

(12)

où R′ à $R_{12}$ et X ont la signification ci-dessus indiquée.

7. Procédé selon les revendications 1 à 6, où on utilise comme agent de condensation faiblement basique du carbonate de sodium ou du carbonate de potassium.

8. Procédé selon les revendications 1 à 7, où on utilise comme solvant inerte dans les conditions de la réaction un solvant aprotique, apolaire ou dipolaire.

9. Procédé selon la revendication 8, où on utilise comme solvant le diméthylformamide.